# EUROPEAN PATENT APPLICATION

(11) **EP 3 733 590 A1**
(43) Date of publication of application: **04.11.2020**
(21) Application number: 20169703.4
(22) Date of filing: 15.04.2020
(51) Int. Cl.: B67D 3/04, A61M 39/00, B65D 47/00

(54) **VALVE ADAPTOR**

(30) Priority: 01.05.2019 GB 201906112
(71) Applicant: Autonumis Limited, Stroud, Gloucestershire GL6 8GA (GB)
(72) Inventor: MARCH, Nathanael, Stroud, Gloucestershire GL6 8GA (GB); SHEA-SIMONDS, Duncan, Leicester LE1 5TT (GB)
(74) Representative: Doherty, William

(57) **Abstract**

A valve adaptor (20) is provided which is for engaging a liquid drain conduit (16) of a sealed liquid container (10) prior to connecting a flow control valve (26) thereto. The adaptor (20) comprises an adaptor body (22) including an inlet (22a) and an outlet (22b and a conduit guide portion (30) into which the liquid drain conduit (16) is extendable, the conduit guide portion (30) including a clamp element (38) which is movable to apply a clamping force between the inlet and the outlet to close the liquid drain conduit (16) received therein. The adaptor (20) also includes a conduit-valve connector (24) comprising an insertable portion (50) having a liquid flow bore (52) therethrough, the insertable portion (50) being insertable into the liquid drain conduit (16) when the liquid drain conduit (16) is positioned in the conduit guide portion (30) to thereby sealingly engage the conduit-valve connector (24) and the conduit guide portion (30).

## Description

The present invention relates to a valve adaptor for engaging a liquid drain conduit prior to connecting a flow control valve thereto, and particularly but not necessarily exclusively for bladder bags for bag-in-box containers for beverages, specifically milk. The invention further relates to a valve assembly for a liquid drain conduit, and to a valve engagement system for connecting a valve to any of a plurality of liquid drain conduits having different diameters, as well as a method of attaching a valve to a pre-sealed liquid drain conduit of a sealed liquid container.

Beverages, and in particular dairy and juice liquids, are often stored in sealed bags, typically bladder bags, inside a box, forming a bag-in-box container. The bag has a hot crimp applied to the liquid drain conduit which acts as the exit drain. This prevents leakage of the beverages from the bag, whilst allowing a user a potential access route into the liquid contained therein. Many different bag manufacturers utilise different tube and crimp designs. Various connector products exist, but these are valve- and tube-specific, and are not interchangeable.

In many dispensing applications, particularly for the dispensing of milk for coffee machines, a bladder bag, or bag-in-box, is inserted into a, preferably refrigerated, dispensing machine, which includes a tap feature. The tap is usually provided having a spring-loaded mechanical clamp which pinches the outside of the liquid drain conduit connected to the bag. This allows for different tubes to be utilised in a gravity-fed system, generally placed on a countertop, but not with a system which requires pumping of the liquid. This limits the utility of such arrangements.

Any user wishing to dispense the liquid via a pumping machine would require the bespoke adaptor based on the bag manufacture's valve arrangement. This requires the user to match their dispensing system to that of the manufacturer, usually forcing the user to continue to utilise only the manufacturer's product thereafter.

The present invention seeks to provide an adaptor for engaging a wide variety of proprietary valves with a range of different liquid drain tubes from different manufacturers.

According to a first aspect of the invention, there is provided a valve adaptor for engaging a liquid drain conduit of a sealed liquid container prior to connecting a flow control valve thereto, the valve adaptor comprising: an adaptor body having an inlet and an outlet and including: a conduit guide portion into which the liquid drain conduit is extendable, the conduit guide portion including a clamp element which is movable to apply a clamping force between the inlet and the outlet to close a liquid drain conduit received therein; and a conduit-valve connector comprising an insertable portion having a liquid flow bore therethrough, the insertable portion being insertable into the liquid drain conduit when the liquid drain conduit is positioned in the conduit guide portion to sealingly engage the conduit-valve connector and the adaptor body.

One of the difficulties of existing valve engagement arrangements is that, once the fluid pressure is released, any adaptor is dislodged from the end of the liquid drain conduit. Some arrangements have overcome this by using sterile needle configurations which puncture the beverage container. The advantage of the present system, however, is that the engagement between the liquid drain conduit and the adaptor is very simple, and no complex sterile apparatus is required. Additionally, the adaptor is capable of providing generic engagement between liquid drain conduits and proprietary valves of many different dimensions, greatly reducing the user's reliance on any particular manufacturer.

Optionally, the insertable tube portion of the conduit-valve connector may include an outer screw thread.

Screw-threaded engagement between the conduit-valve connector and the liquid drain conduit is the simplest means by which a tight seal can be created, since the screw-threading readily deforms the material of the liquid drain conduit against the inner surfaces of the engagement throat.

Preferably, the adaptor body may comprise an engagement throat adjacent to the conduit guide portion against which the conduit-valve connector sealingly engages.

The engagement throat provides a substantial element against which the insertable portion and liquid drain conduit can bear, once deformed, to provide frictional engagement between the adaptor body and conduit-valve connector.

The engagement throat may include at least one anti-rotation element, and the or each anti-rotation element may comprise a longitudinal rib in the engagement throat.

One or more anti-rotation elements limit the risk of twisting of the liquid drain conduit within the adaptor body, preventing blockages or flow limitations within the liquid drain conduit which could hamper normal operation.

Preferably, the clamp element may be formed as a movable arm having an inwardly-facing projection for providing the clamping force.

A movable arm allows for an easy clamping force to be applied by a user to cut off flow through the liquid drain conduit whilst the adaptor is being engaged therewith.

Said inwardly-facing projection may be dimensioned to apply the clamping force spaced apart from a central axis of the adaptor body, to thereby crimp the liquid drain conduit when the clamping force is applied.

Providing a crimp within the conduit guide portion provides a more effective blockage to liquid flow therethrough, decreasing the likelihood of liquid leakage whilst the adaptor is being attached.

The movable arm may preferably be formed as a cantilevered arm.

A cantilever arrangement allows the user to apply the clamping force to the liquid drain conduit using only one hand, leaving their other hand free for alignment and/or engagement of the conduit-valve connector.

Optionally, the conduit guide portion may include an internal clamping member against which the clamping force is applied by the inwardly-facing projection.

A dedicated internal clamping member ensures that the correct clamping force can be applied without risking damage to the liquid drain conduit during the clamping process.

Preferably, the conduit-valve connector may further comprise a cap.

The cap provides a simple handle for attachment of the conduit-valve connector to the adaptor body, allowing for easy rotation thereof to provide the screwing action.

In a preferable embodiment, the cap may include a screw-thread for engaging with a flow control valve for the liquid drain conduit.

Screw-threaded engagement will allow for ready connection between the cap and existing valve arrangements. Many proprietary valves utilise screw threaded connectors, and therefore the cap can be fashioned accordingly.

The valve adaptor may further comprise clamp locking means for locking the clamping element so as to maintain the clamping force.

Whilst a user can manually hold the clamping force in place, it is preferred that a locking means be provided so that the user can then use both hands to assist with the connection of the conduit-valve connector to the adaptor body.

Optionally, the clamp locking means may comprise a two-part latch between a radially-projecting portion of the conduit guide portion and the clamping element.

A latching mechanism provides a simple means of urging the movable arm both into the clamped and non-clamped configurations, preventing accidental release of the clamping force during attachment of the conduit-valve connector.

Preferably, the two-part latch may comprise a projection extending from the radially-projecting portion and a latching extension of the clamping element, the projection and latching extension being laterally offset relative to one another.

This arrangement has the advantage of preventing disengagement of the movable arm during any twisting engagement between the adaptor body and the conduit-valve connector.

The radially-projecting portion may form a gripping platform for a user.

An outer projection provides something for the user to grasp to counter the rotational forces being applied by the conduit-valve connector, and significantly reduces the risk of twisting or tearing the liquid drain conduit upstream of the valve adaptor.

At least part of the adaptor body may include a lateral aperture dimensioned to receive the liquid drain conduit therethrough.

A lateral aperture or slot allows for the liquid drain conduit to be introduced from the side. Some liquid drain conduits available have a larger diameter than the internal bore of the collar, and therefore the lateral slot enables the adaptor to be used with such conduits.

Preferably, the conduit-valve connector may comprise a head portion at one end of the insertable portion which has a greater width than the insertable portion.

It may be foreseeable that the sealing effectiveness is decreased if the insertable portion of the conduit-valve connector is over-inserted. However, the shape of the conduit-valve connector can be made such that over-insertion is not possible.

The adaptor body may further comprise a conduit receiving collar at or adjacent to an end of the conduit guide portion which is receivable around the liquid drain conduit.

A collar which is fully receivable around the liquid drain conduit may simplify the attachment of the adaptor body, since there is less risk of the adaptor body falling off the liquid drain conduit prior to engagement. It may also serve to ensure correct alignment of the clamp with respect to the engagement throat.

According to a second aspect of the invention, there is provided a valve assembly for a liquid drain conduit of a sealed liquid container, the valve engagement assembly comprising a valve adaptor in accordance with the first aspect of the invention, and a flow control valve connected to the conduit-valve connector.

It is preferred that the flow control valve be provided with the valve adaptor when attaching the adaptor, since this negates the need to provide subsequent engagement.

According to a third aspect of the invention, there is provided a valve engagement system for connecting a valve to any of a plurality of liquid drain conduits having different diameters, the valve engagement system comprising: an adaptor body having an inlet and an outlet and including: a conduit guide portion into which the liquid drain conduit is extendable, the conduit guide portion including a clamp element which is movable to apply a clamping force between the inlet and the outlet to close a liquid drain conduit received therein; and a plurality of conduit-valve connectors, each conduit-valve connector comprising an insertable portion of a different diameter having a liquid flow bore therethrough, the insertable portion being insertable into one of the plurality of liquid drain conduits when said liquid drain conduit is positioned in the conduit guide portion to sealingly engage conduit-valve connector and the adaptor body.

The ability to attach the valve adaptor to a wide variety of different liquid drain conduits and/or proprietary valves significantly increases the utility of user's existing flow control systems, since they are no longer locked to a specific bespoke arrangement.

According to a fourth aspect of the invention, there is provided a method of attaching a valve to a pre-sealed liquid drain conduit of a sealed liquid container, the method comprising the steps of: a] providing a valve adaptor in accordance with the first aspect of the invention; b] feeding the liquid drain conduit into the conduit guide portion; c] moving the clamp element to apply the clamping force to close and crimp the liquid drain conduit; d] cutting an overhanging end of the liquid drain conduit extending from the conduit guide portion; e] inserting the conduit-valve connector into the cut end of the liquid drain conduit, so as to deform the liquid drain conduit to sealingly engage the conduit-valve connector and adaptor body, the conduit-valve connector either being engaged with or engagable with a valve to be connected to the liquid drain conduit; and f] moving the clamp element to release the clamping force to permit liquid flow between the sealed liquid container and the valve.

Optionally, the sealed liquid container may be a beverage container, such as a bag for a bag-in-box.

According to a fifth aspect of the invention, there is provided an adaptor body for a valve adaptor for engaging a liquid drain conduit of a sealed liquid container prior to connecting a flow control valve thereto, the adaptor body comprising: and inlet and an outlet, and a conduit guide portion into which the liquid drain conduit is extendable, the conduit guide portion including a clamp element which is movable to apply a clamping force between the inlet and the outlet to close a liquid drain conduit received therein.

The invention will now be more particularly described, by way of example only, with reference to the accompanying drawings, in which:
Figure 1 shows a plan view of one embodiment of a sealed bladder bag for a bag-in-box, shown empty of fluid, and having a pre-crimped liquid drain conduit attached thereto, as known in the art;
Figure 2 shows a side perspective view of one embodiment of a valve adaptor in accordance with the first aspect of the invention, connected to a flow control valve to be attached to a liquid drain conduit;
Figure 3 shows a side perspective representation of the adaptor body of the valve adaptor shown in Figure 2;
Figure 4 shows an enlarged perspective view of the engagement throat of the valve adaptor shown in Figure 2;
Figure 5 shows a side view of the adaptor body of the valve adaptor of Figure 2, with the liquid drain conduit inserted therein and being clamped by the clamping element;
Figure 6 shows the side view of the adaptor body of the valve adaptor of Figure 5, following cutting of the hot crimped end of the liquid drain conduit, and aligned with the conduit-valve connector and flow control valve; and
Figure 7 shows an enlarged side view of the engagement between the conduit-valve connector, with the cap shown in cross-section for clarity, the liquid drain conduit, and engagement throat of the valve adaptor.

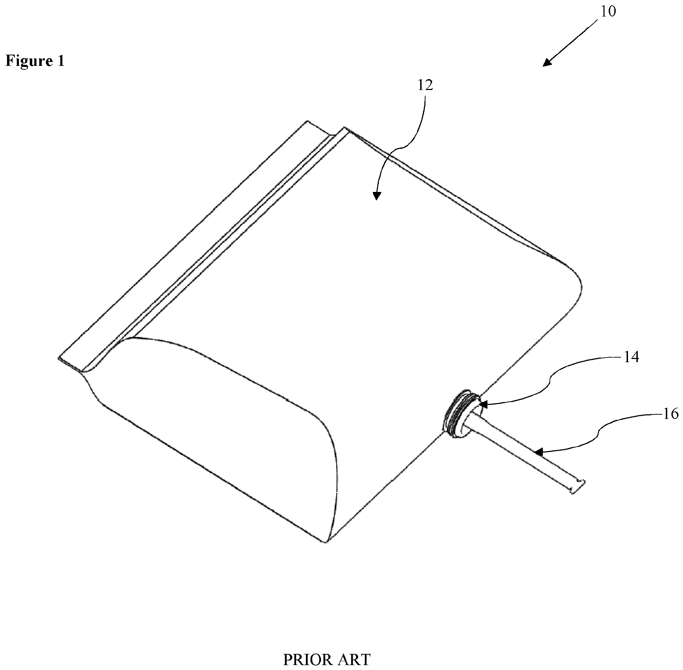

Referring to Figure 1, there is indicated a bag for a bag-in-box, formed as a bladder bag, referenced globally at 10, which is common in the art. The bladder bag 10 comprises a fluid bladder 12 having a port 14 from which a liquid drain conduit 16 extends. Typically, the port 14 would be provided with a sealing cap and pre-crimped liquid drain conduit 16. The challenge is therefore to readily engage a flow control valve to the liquid drain conduit 16 in a generalised manner for a wide variety of different valves, without resulting in leakage.

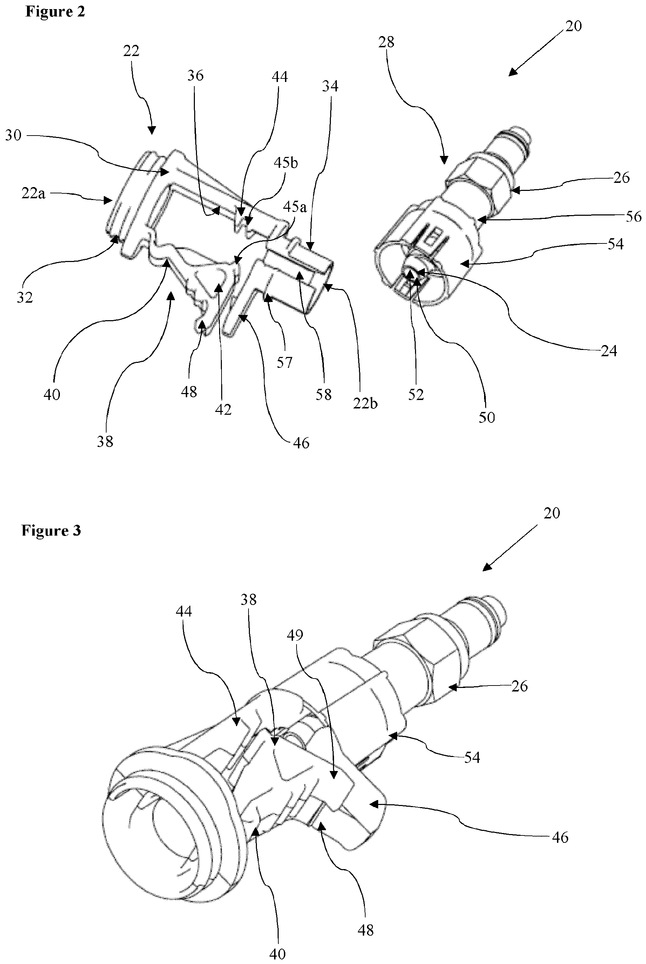

The valve adaptor 20 of the present invention is shown in Figure 2. There is an adaptor body 22 having an inlet 22a and an outlet 22b and which is receivable around the liquid drain conduit 16, and a conduit-valve connector 24 which is engagable with both the adaptor body 22 and the liquid drain conduit 16, and with which, the flow control valve 26 is engaged, releasably or otherwise, to thereby form a valve assembly 28.

The adaptor body 22 comprises a conduit guide portion 30 into which the liquid drain conduit 16 is extendable, preferably including a conduit receiving collar 32 which is formed as a complete ring. At one end of the conduit guide portion 30, there is an engagement throat 34, via which the conduit-valve connector 24 engages with the adaptor body 22.

The conduit guide portion 30 may have an elongate wall 36 against which the conduit can rest when the liquid drain conduit 16 is inserted therein. The conduit guide portion 30 further includes a clamp element 38 which is movable to apply a clamping force at and/or into the conduit guide portion 30 to close the liquid drain conduit 16 when inserted therein. As such, the conduit guide portion 30 is formed so as to have a void into which the clamp element 38 is movable.

The clamp element 38 is here formed as a movable, preferably cantilevered, arm 40 having an inwardly-facing, preferably triangular, projection 42 for providing the clamping force. The conduit guide portion 30 may also include an internal clamping member 44 against which the clamping force is applied by the inwardly-facing projection 42. This creates a hammer-and-anvil type of clamping action to apply a crimp to the liquid drain conduit 16 between the inlet 22a and the outlet 22b, and is therefore differentiated from, for example, an end valve unit. The inwardly facing projection 42 may have a dedicated crimp-forming member 45a or similar portion which creates an enhanced clamping force at the tip of the inwardly-facing projection. The internal clamping member 44 may then have a shape-mated receiver 45b, the crimp-forming member 45a and receiver 45b thereby forming the crimping mechanism.

In a rest condition, the clamp element 38 may rest against a radially-projecting portion 46 of the conduit guide portion 30, which is at or adjacent to the engagement throat 34.

This may serve to act as a gripping platform for a user's finger when either the conduit-valve connector 24 is being applied, or the clamp element 38 is engaged.

An outer nub or projection 48 may be provided on the radially-projecting portion 46, preferably formed as a triangular wedge which tapers outwardly in the radial direction, which forms part of a two-part latch with the clamp element 38 to thereby create a clamp locking means for holding the clamp element 38 in the correct configuration. At rest, an outer edge of the clamp element 38 will contact the tapering surface of the projection 48, and the clamp element 38 will naturally be urged out of the clamping condition. However, if a user applies sufficient force onto the clamping element 38, the outer edge of the clamp element 38 will push past the tapering surface of the projection 48, instead contacting a flat edge which is perpendicular to the radially-projecting portion 46, and inhibiting movement of the clamp element 38. This clamped configuration can be seen in Figure 2. The projection 48 prevents the arm 40 of the clamp element 38 from disengaging by means of any twisting action applied during engagement of the conduit-valve connector 24.

The projection 48 is indicated in more detail in Figure 3. There is a latching extension 49 which is positioned so as to outwardly extend from the clamp element 38 at or adjacent to the radially-projecting portion 46, and the projection 48 is laterally offset relative to the latching extension 49. The lateral offset is matched to the direction of rotational engagement between the adaptor body 22 and the conduit-valve connector 24, such that, upon engagement therebetween, any twisting motion urges the latching extension 49 against the projection 48. This advantageously improves the locking of the clamp element 38 in position, and the twisting action does not result in accidental disengagement of the clamp element 38 with the liquid drain conduit 16.

The conduit-valve connector 24 is formed so as to be receivable into a cut end of the liquid drain conduit 16. A plurality of different dimensions of conduit-valve connector 24 may be required, based on the dimensions of different proprietary liquid drain conduits 16. The conduit-valve connector 24 has an insertable portion 50 having a liquid bore 52 therethrough. The insertable portion 50 is preferably integrally formed with the conduit-valve connector 24, but could be a demountably engagable component. A valve assembly 28 can therefore be constructed with the conduit-valve connector 24. A cap 54 is provided which fits around the conduit-valve connector 24, and improves the ease with which the conduit-valve connector 24 can be engaged with the liquid drain conduit 16. The cap 54 may then also include a screw-threaded portion 56, typically formed as the neck of the cap 54, which is engagable with the flow control valve 26 to be attached to the liquid drain conduit 16. The cap 54 itself may be snap-fit engagable with the adaptor body 22, for example, via a circumferential ridge 57 on the engagement throat 34. It will, however, be appreciated that alternative connection methods could be considered to permit engagement of the flow control valve 26. For instance, the flow control valve 26 could be provided as an integral component of the conduit-valve connector 24, or could be provided having a snap-fit engagement instead.

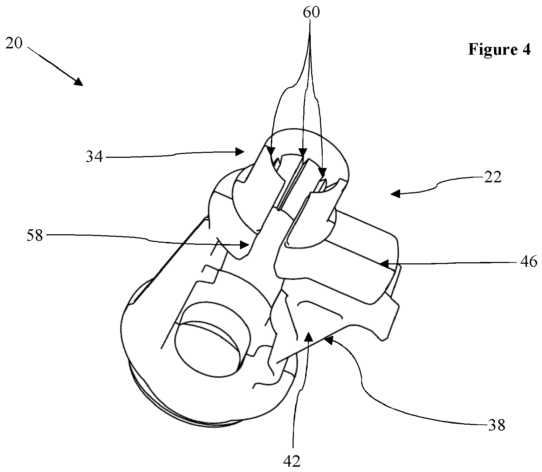

The engagement throat 34 is shown in detail in Figure 4. It is preferably dimensioned so that the liquid drain conduit 16 is snugly but easily insertable therein, prior to engagement of the conduit-valve connector 24. The engagement throat 34 is formed as a cylinder wall having a longitudinal access aperture 58 therein. This allows the liquid drain conduit 16 to be introduced from the side.

It is preferred that the engagement throat 34 include one or more anti-rotation elements, such as longitudinal ribs 60. These serve to prevent rotation of the liquid drain conduit 16 when the conduit-valve connector 24 is being connected.

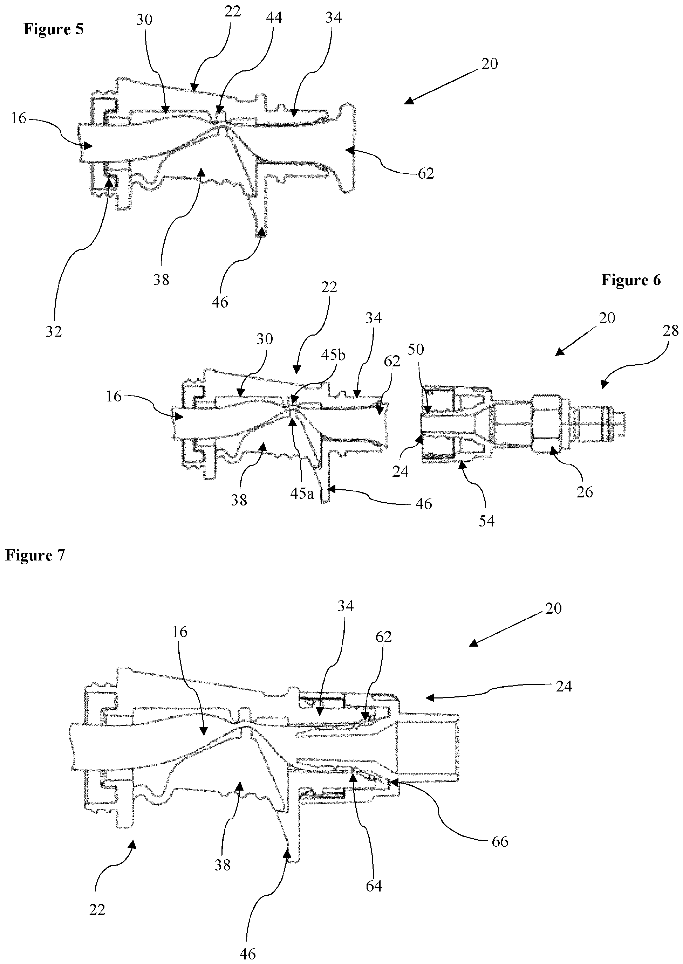

Figure 5 shows the first step of use of the valve adaptor 20. The liquid drain conduit 16 is inserted through the conduit-receiving collar 32 and conduit guide portion 30 and engagement throat 34. The heat-sealed end 62 of the liquid drain conduit 16 is positioned so as to protrude slightly from the end of the engagement throat 34.

The clamp element 38 is then engaged by the user, preferably being held in place by a locking means, though it could alternatively be manually held by the user during the process. The inwardly-facing projection 42 of the clamp element 38 is dimensioned to apply the clamping force spaced apart from an axis of the engagement throat 34, to thereby crimp the liquid drain conduit 16 when the clamping force is applied, in particular by shape-mating engagement of the crimp-forming member 45a and the shape-mating receiver 45b. This creates an effective closure to prevent leakage of liquid from the liquid drain conduit 16 during the adaptor application process.

As shown in Figure 6, the heat-sealed end 62 of the liquid drain conduit 16 is then cut, and the insertable portion 50 of the conduit-valve connector 24 inserted into the now open end 62. This is typically achieved by rotating the valve assembly 28.

The conduit-valve connector 24 is shown in more detail in Figure 7, to better highlight the connection method. The insertable portion 50 of the conduit-valve connector 24 has an outer screw thread 64 which deforms the material of the liquid drain conduit 16 when the conduit-valve connector 24 is screwed into place. The conduit-valve connector 24 comprises a stop portion 66 at one end of the insertable portion 50 which has a greater width than the insertable portion 50. This may serve to prevent over-insertion of the insertable portion into the liquid drain conduit 16.

This process is all performed whilst the open end 62 of the liquid drain conduit 16 is received within the engagement throat 34, being snugly received therein. The deformation of the material of the liquid drain conduit by the screw thread 64 thereby forms a seal between the engagement throat 34 and the outer surface of the liquid drain conduit 16. The anti-rotation elements help then to prevent twisting of the liquid drain conduit 16 within the engagement throat 34. The projection 48 also prevents disengagement of the clamp element 38 as a result of the twisting action, so that the liquid drain conduit 16 remains clamped throughout the engagement process.

The provision of the seal is sufficient to prevent ejection of the conduit-valve connector 24 from the liquid drain conduit 16 once the clamp element 38 is released and a backpressure of liquid is recovered.

The method of attaching a valve 26 to a pre-sealed liquid drain conduit 16 of a sealed liquid container 10 in accordance with the present disclosure can therefore be summarised as follows: the valve adaptor 20 is provided, and the liquid drain conduit 16 is fed through the conduit guide portion 30 and engagement throat 34, and preferably also through the conduit-receiving collar 32. The clamp element 38 is moved to apply the clamping force to close and crimp the liquid drain conduit 16, and an overhanging end 62 of the liquid drain conduit 16 extending from the engagement throat 34 is cut. The conduit-valve connector 24 is then inserted into the cut end 62 of the liquid drain conduit 16, so as to deform the liquid drain conduit 16 to form a seal between the conduit-valve connector 24 and engagement throat 34, the conduit-valve connector 24 either being engaged with or engagable with a valve 26 to be connected to the liquid drain conduit 16. The clamp element 38 can then be moved to release the clamping force to permit liquid flow between the sealed liquid container 10 and the valve 26.

The present valve adaptor 20 allows for the creation of a valve engagement system for connecting a valve to any of a plurality of liquid drain conduits 16 having different diameters. In such a system, there is provided a single adaptor body 22, and a plurality of conduit-valve connectors 24. Each conduit-valve connector 24 comprises an insertable portion 50 of a different diameter having a liquid flow bore 52 therethrough, the insertable portion 50 being insertable into one of the plurality of liquid drain conduits 16 when said liquid drain conduit 16 is positioned in the engagement throat 34 to thereby create a seal between the conduit-valve connector 24 and the engagement throat 34.

Whilst screw-threaded engagement between the conduit-valve connector and the liquid drain conduit is discussed and preferred, it will be appreciated that the necessary requirement is that the conduit-valve connector sufficiently deform the liquid drain conduit to form a seal with the engagement throat when engaged. A ribbed push-fit engagement, or similar deforming process, could be considered as an alternative to screw-threaded engagement. Similarly, whilst the use of the engagement throat as a means of providing resistance against the insertable portion of the conduit-valve connector is preferred, the engagement throat may be dispensed with and the conduit-valve connector may be able to be held in place purely by deformation for low-pressure arrangements.

The present invention is not solely suitable for bags for bag-in-boxes. Bag-in-box beverage containers could also be considered, as indeed could other beverage containers having liquid drain conduits which are suitably deformable, such as short-term kegs. In particular, liquid drain conduits may be found, and therefore adaptors used with, drinking yoghurt or edible oil containers. The present invention is therefore not intended to be limited purely to use with bladder bags, or pergal bags as they are sometimes referred to, as used in the dairy industry.

It is therefore possible to provide a valve adaptor for liquid drain conduits of beverage containers, which allows for proprietary valves to be connected to beverage containers with which they are not otherwise compatible. This reduces the user's reliance on a single manufacturer for all of their equipment needs, significantly reducing the overall equipment cost. The engagement of the valve with the beverage container may also be much simplified compared with existing products which are designed to be used with only one type of beverage container. The valve adaptor allows the user to interchange between liquid drain conduits of different dimensions and proprietary valves of different types, without rendering their entire existing set-up obsolete.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein.

## Claims

1. A valve adaptor (20) for engaging a liquid drain conduit (16) of a sealed liquid container (10) prior to connecting a flow control valve (26) thereto, the valve adaptor (20) comprising:
an adaptor body (22) having an inlet (22a) and an outlet (22b) and including a conduit guide portion (30) into which the liquid drain conduit (16) is extendable, the conduit guide portion (30) including a clamp element (38) which is movable to apply a clamping force between the inlet (22a) and the outlet (22b) to close a liquid drain conduit (16) received therein; and
a conduit-valve connector (24) comprising an insertable portion (50) having a liquid flow bore (52) therethrough, the insertable portion (50) being insertable into the liquid drain conduit (16) when the liquid drain conduit (16) is positioned in the conduit guide portion (30) to sealingly engage the conduit-valve connector (24) and the adaptor body (22).

2. A valve adaptor (20) as claimed in claim 1, wherein the insertable portion (50) of the conduit-valve connector (24) includes an outer screw thread.

3. A valve adaptor (20) as claimed in claim 1 or claim 2, wherein the adaptor body (22) comprises an engagement throat (34) adjacent to the conduit guide portion (30) against which the conduit-valve connector (24) sealingly engages.

4. A valve adaptor (20) as claimed in claim 3, wherein the engagement throat (34) includes at least one anti-rotation element (60).

5. A valve adaptor (20) as claimed in any one of the preceding claims, wherein the clamp element (38) is formed as a movable arm (40) having an inwardly-facing projection (42) for providing the clamping force.

6. A valve adaptor (20) as claimed in claim 5, wherein the inwardly-facing projection (42) is dimensioned to apply the clamping force spaced apart from a central axis of the adaptor body (22), to thereby crimp the liquid drain conduit (16) when the clamping force is applied.

7. A valve adaptor (20) as claimed in claim 5 or claim 6, wherein the conduit guide portion (30) includes an internal clamping member (44) against which the clamping force is applied by the inwardly-facing projection (42).

8. A valve adaptor (20) as claimed in any one of the preceding claims, wherein the conduit-valve connector (24) further comprises a cap (54), wherein the cap (54) includes a screw-thread (56) for engaging with a flow control valve (26) for the liquid drain conduit (16).

9. A valve adaptor (20) as claimed in any one of the preceding claims, further comprising a two-part latch between a radially-projecting portion (46) of the conduit guide portion (30) and the clamping element (38).

10. A valve adaptor (20) as claimed in claim 9, wherein the two-part latch comprises a projection (48) extending from the radially-projecting portion (46) and a latching extension (49) of the clamping element (38), the projection (48) and latching extension (49) being laterally offset relative to one another.

11. A valve adaptor (20) as claimed in any one of the preceding claims, wherein at least part of the adaptor body (22) includes a lateral aperture (58) dimensioned to receive the liquid drain conduit (16) therethrough.

12. A valve assembly for a liquid drain conduit (16) of a sealed liquid container (10), the valve assembly comprising a valve adaptor (20) as claimed in any one of the preceding claims, and a flow control valve (26) connected to the conduit-valve connector (26).

13. A valve engagement system for connecting a valve to any of a plurality of liquid drain conduits having different diameters, the valve engagement system comprising:
an adaptor body (22) having an inlet (22a) and an outlet (22b) and including a conduit guide portion (30) into which the liquid drain conduit (16) is extendable, the conduit guide portion (30) including a clamp element (38) which is movable to apply a clamping force between the inlet (22a) and the outlet (22b) to close a liquid drain conduit (16) received therein; and
a plurality of conduit-valve connectors (24), each conduit-valve connector (24) comprising an insertable portion (50) of a different diameter having a liquid flow bore therethrough, the insertable portion (50) being insertable into one of the plurality of liquid drain conduits (16) when said liquid drain conduit (16) is positioned in the conduit guide portion (30) to sealingly engage conduit-valve connector (24) and the adaptor body (22).

14. A method of attaching a valve to a pre-sealed liquid drain conduit (16) of a sealed liquid container (10), the method comprising the steps of:
a] providing a valve adaptor (20) as claimed in any one of claims 1 to 11;
b] feeding the liquid drain conduit (16) into the conduit guide portion (30);
c] moving the clamp element (38) to apply the clamping force to close and crimp the liquid drain conduit (16);
d] cutting an overhanging end (62) of the liquid drain conduit (16) extending from the conduit guide portion (30);
e] inserting the conduit-valve connector (24) into the cut end of the liquid drain conduit (16), so as to deform the liquid drain conduit (16) to sealingly engage the conduit-valve connector (24) and adaptor body (22), the conduit-valve connector (24) either being engaged with or engagable with a valve to be connected to the liquid drain conduit (16); and
f] moving the clamp element (38) to release the clamping force to permit liquid flow between the sealed liquid container (10) and the valve.

15. An adaptor body (22) for a valve adaptor (20) for engaging a liquid drain conduit (16) of a sealed liquid container (10) prior to connecting a flow control valve (26) thereto, the adaptor body (22) comprising:
an inlet (22a) and an outlet (22b), and a conduit guide portion (30) into which the liquid drain conduit (16) is extendable, the conduit guide portion (30) including a clamp element (38) which is movable to apply a clamping force between the inlet (22a) and the outlet (22b) to close the said liquid drain conduit (16) received therein.
